# EUROPÄISCHE PATENTANMELDUNG

(11) **EP 0 548 020 A2**
(43) Veröffentlichungstag der Anmeldung: **23.06.1993**
(21) Anmeldenummer: 92810975.0
(22) Anmeldetag: 10.12.1992
(51) Int. Cl.: G01N 33/00, G01N 31/00, G01N 27/416

(54) **Verfahren zur kontinuierlichen, quantitativen Bestimmung von fluorhaltigen Verbindungen**

(30) Priorität: 19.12.1991 CH 3778/91
(71) Anmelder: CIBA-GEIGY AG, CH-4002 Basel (CH)
(72) Erfinder: Frenk, Klaus, W-7860 Schopfheim 2 (DE)

(57) **Zusammenfassung**

Verfahren zur kontinuierlichen, quantitativen Bestimmung von fluorhaltigen Verbindungen in Gasen, dadurch gekennzeichnet, dass man das die fluorhaltige Verbindung enthaltende Gas mit einer wässrigen Lösung eines Amins behandelt und die Konzentration der abgespaltenen Fluoridionen mit einer direktpotentiometrischen Methode bestimmt.

## Beschreibung

Die Erfindung betrifft ein Verfahren zur kontinuierlichen, quantitativen Bestimmung von fluorhaltigen Verbindungen in Gasen und ein Mittel zur Freisetzung von Fluoridionen aus fluorhaltigen Verbindungen.

Im Zuge einer genaueren Überwachung der Abluft, aber auch der Umgebungsluft, bei chemischen Prozessen, besteht ein steigendes Bedürfnis zur kontinuierlichen und quantitativen Bestimmung von fluorhaltigen Verbindungen in Gasen. Schwierigkeiten bereitet hierbei insbesondere die geringe Konzentration der fluorhaltigen Verbindungen in den zu untersuchenden Gasen, vorzugsweise Luft, und die ungenügende "Hydrolyse" der fluorhaltigen Verbindungen zur Abspaltung und Freisetzung der Fluoridionen.

Gegenstand der Erfindung ist somit ein Verfahren zur kontinuierlichen, quantitativen Bestimmung von fluorhaltigen Verbindungen in Gasen, dadurch gekennzeichnet, dass man das Gas, das die fluorhaltige Verbindung enthält, mit einer wässrigen Lösung eines Amins behandelt und die Konzentration der abgespaltenen Fluoridionen mit einer direktpotentiometrischen Methode bestimmt.

Mit dem erfindungsgemässen Verfahren gelingt es auch geringste Spuren von fluorhaltigen Verbindungen in Gasen kontinuierlich nachzuweisen.

Im Sinne der vorliegenden Erfindung handelt es sich bei den zu untersuchenden Gasen (Messgas) im allgemeinen um Gasgemische, vor allem Luft oder luftähnliche Gasgemische. Es können jedoch auch Einzelgase, wie z.B. Stickstoff, auf ihren Gehalt an fluorhaltigen Verbindungen untersucht werden.

Besonders geeignet ist das Verfahren zur Bestimmung von organischen Fluorverbindungen und auch von Fluorwasserstoff. Enthalten die organischen Fluorverbindungen mehrere Fluoratome, so genügt prinzipiell die Überführung eines dieser Fluoratome in ein Fluoridion zur genauen quantitativen Bestimmung der Verbindung. Der Grad der Überführung lässt sich leicht mit einem Kalibrierexperiment feststellen, bei dem man eine definierte Menge der fluorhaltigen Verbindung einem Gasstrom zusetzt und das erfindungsgemässe Verfahren durchführt. Ganz besonders geeignet ist das Verfahren zur Bestimmung von Fluorwasserstoff und fluorhaltigen organischen Verbindungen, bei denen das Fluoratom direkt an einen aromatischen Ring gebunden ist. Zusätzliche aktivierende Gruppen, wie die Nitro-, Carboxy- oder Cyanogruppe, die einen -M-Effekt aufweisen, erhöhen die Fluoridionenausbeute. Die zu bestimmenden fluorhaltigen organischen Verbindungen sind vorzugsweise bei Temperaturen von 20-35°C und unter Normaldruck flüssig oder gasförmig. Zur Erhöhung der Fluoridionenausbeute ist es möglich, während der Behandlung mit dem Amin, bei höheren Temperaturen zu arbeiten. Beispiele für fluorhaltige organische Verbindungen sind Fluorbenzol, 2-, 3-, und 4-Fluortoluol, 4,4'-Difluordiphenylmethan, 1,3- und 1,4-Difluorbenzol, 2,6-Difluorbenzonitril, 4,4'-Difluorbenzophenon, Hexafluorbenzol, sowie fluorierte Pyridine, Pyrimidine und Triazine, wobei 2,4,6-Trifluor-1,3,5-triazin hervorzuheben ist.

Die Konzentration der fluorhaltigen Verbindung im Gas liegt typischerweise in einem Bereich von 0,01 bis 10 mg/m³ (berechnet als Fluorwasserstoff).

Die Behandlung des Messgasstromes, dessen Konzentration an fluorhaltiger Verbindung bestimmt werden soll, mit der wässrigen Lösung eines Amins (Absorptionslösung) erfolgt beispielsweise durch Verdüsung der Absorptionslösung im Messgasstrom. Bevorzugt ist die Verdüsung quer zur Strömungsrichtung des Messgases. Das Verhältnis zwischen Messgasstrom und Absorptionslösungsstrom kann in einem weiten Bereich liegen, günstig sind Verhältnisse von 1000:1 bis 50000:1, wobei ein Verhältnis von etwa 20000:1 besonders bevorzugt ist. Es ergibt sich so z.B. für einen Messgasstrom von 400 l/h ein Absorptionslösungsstrom von 20 ml/h bei der besonders bevorzugten Ausführungsform.

Die wässrige Lösung enthält das Amin üblicherweise in einer Menge von 0,01 bis 1 mol/l, wobei ein Bereich von 0,05 bis 0,3 mol/l besonders bevorzugt ist.

Im Rahmen der Erfindung geeignete Amine sind sowohl Mono-, Di- wie auch Poly-C₃-C₂₀-Amine. Die genannten Amine sind nicht auf geradkettige beschränkt, sondern schliessen verzweigtkettige und weiter substituierte Amine ein. Es können sowohl aliphatische als auch aromatische Amine verwendet werden. Bevorzugt verwendet man primäre oder sekundäre Amine. Weiterhin bevorzugt sind aliphatische Amine, welche auch als heterocyclische Verbindungen vorliegen können, wobei der Heterocyclus substituiert sein kann und/oder wobei ein weiteres Heteroatom sich im Ring befinden kann. Beispielhaft seien Diethylentriamin, Prolin, Morpholin, Piperazin oder Phenylendiamin genannt. Besonders bevorzugt ist Piperazin.

Neben dem Amin kann die wässrige Lösung auch noch weitere Stoffe enthalten, die, bedingt durch die gewählte direktpotentiometrische Methode, zur Kalibrierung der Messeinrichtung oder zu deren Optimierung dienen. So ist es insbesondere vorteilhaft den pH-Wert der Lösung den vorzugsweise verwendeten ionenselektiven Elektroden anzupassen. Üblicherweise arbeiten fluoridsensitive Elektroden in einem Bereich von pH 6±0,5 weitgehend linear. Die Einstellung des pH-Wertes erfolgt beispielsweise durch den Zusatz von Mineralsäuren, wie Salzsäure, Bromwasserstoffsäure oder Schwefelsäure. Weiter kann der wässrigen Lösung ein Elektolyt, beispielsweise Kochsalz, eine geringe Menge einer fluorhaltigen Referenzverbindung, beispielsweise 10⁻⁴ mol/l Natriumfluorid, oder ein Tensid zugesetzt werden.

Das direktpotentiometrische Messsystem besteht bevorzugt aus einer fluorionensensitiven Elektrode und einer Referenzelektrode, sowie einer Ausgabeeinheit, welche die Spannungswerte in die Konzentrationen der fluorhaltigen Verbindungen im Messgasstrom umrechnet.

Eine bevorzugte Ausführung des erfindungsgemässen Verfahrens besteht in der Verwendung eines "Compur Ionotox Monitors®" (Bayer Diagnostic), in welchem man als Absorptionslösung eine wässrige Lösung eines Amins verwendet. Die oben angegebenen Bevorzugungen hinsichtlich des Amins gelten analog.

Ein weiterer Gegenstand der Erfindung ist ein Mittel zur Freisetzung von Fluoridionen aus fluorhaltigen Verbindungen enthaltend 0,01-1 mol/l eines Amins, eine geringe Menge einer fluorhaltigen Referenzverbindung und Wasser, wobei der pH-Wert der Lösung in einem Bereich von 6±0,5 liegt. Analog den obigen Angaben für das Amin und die weiteren Zusatzstoffe gelten die dort beschriebenen Bevorzugungen auch für das erfindungsgemässe Mittel. Besonders bevorzugt ist ein Mittel enthaltend neben Wasser und 0,05 bis 0,3 mol/l Piperazin, etwa 10⁻⁴ mol/l Natriumfluorid, dessen pH-Wert mit Salzsäure auf 5,8 eingestellt ist.

Ein weiterer Gegenstand der Erfindung ist die Verwendung einer Lösung enthaltend 0,01-1 mol/l eines Amins zur Freisetzung von Fluoridionen aus einer fluorhaltigen Substanz. Die erfindungsgemässe Lösung kann weitere Bestandteile haben, insbesondere die oben im Zusammenhang mit der Beschreibung des Verfahrens genannten, und weist die dort beschriebenen Bevorzugungen auf.

Durch das erfindungsgemässe Verfahren bzw. die Verwendung des erfindungsgemässen Mittels erfolgt eine schnelle und weitgehende Hydrolyse von fluorhaltigen Verbindungen. Es wird so möglich, eine kontinuierliche und quantitative Analyse eines Gasstromes auf fluorhaltige Verbindungen durchzuführen.

Ein Anwendung des erfindungsgemässen Verfahrens zur quantitativen Bestimmung des Gehaltes an chlor-, brom-. oder iodhaltigen Verbindungen ist ebenfalls möglich. So kann etwa Cyanurchlorid in gleicher Weise bestimmt werden.

Die folgenden Beispiele veranschaulichen die Erfindung, ohne sie jedoch auf die dort angegebenen Verfahrensparameter beschränken zu wollen.

### Beispiel 1:

In einem "Compur Ionotox Monitor®" (Bayer Diagnostic) wird als Absorptionslösung eine wässrige Lösung von:
. 0,1 mol/l Piperazin,
. 0,15 mol/l HCl und
. 1·10⁻⁴ mol/l NaF

verwendet. Es wird bei 400 l/h Messgasstrom und 20 ml/h Absorptionslösungsstrom gemessen.

Bei der Bestimmung eines Gasstromes enthaltend 1 ppm 2,4,6-Trifluor-1,3,5-triazin erreicht man mit der Absorptionslösung die praktisch vollständige Hydrolyse eines Fluoratomes, das als Fluoridion potentiometrisch quantitativ bestimmt wird.

### Beispiel 2 :

In einem "Compur Ionotox Monitor®" (Bayer Diagnostic) wird als Absorptionslösung eine wässrige Lösung verwendet, die 0,2 mol/l Morpholin und 1·10⁻⁴ mol/l NaF enthält und deren pH-Wert mittels HBr auf 6±0,5 eingestellt wird. Die Menge an freigesetzten Fluoridionen wird bei 200 l/h Messgasstrom und 15 ml/h Absorptionslösungsstrom gemessen.

Als Messgas dient ein N₂-Gasstrom, der variable Mengen an 4,4'-Difluordiphenylmethan (0,5-5 ppm) enthält. Die gemessene Menge an freigesetzten Fluoridionen wird kontinuierlich potentiometrisch bestimmt.

### Beispiele 3 :

In einem "Compur Ionotox Monitor®" (Bayer Diagnostic) wird als Absorptionslösung eine wässrige Lösung verwendet, die 0,2 mol/l Piperazin und 1·10⁻⁴ mol/l NaF enthält und deren pH-Wert mittels H₂SO₄ auf 6±0,5 eingestellt wird. Die Menge an freigesetzten Fluoridionen wird bei 600 l/h Messgasstrom und 20 ml/h Absorptionslösungsstrom gemessen.

Als Messgas dient ein Gasstrom aus Umgebungsluft, der variable Mengen an 2,6-Difluorbenzonitril (0,5-5 ppm) enthält. Die gemessene Menge an freigesetzten Fluoridionen wird kontinuierlich potentiometrisch bestimmt.

### Beispiele 4 :

In einem "Compur Ionotox Monitor®" (Bayer Diagnostic) wird als Absorptionslösung eine wässrige Lösung verwendet, die 0,1 mol/l Diethylentriamin und 1·10⁻⁴ mol/l NaF enthält und deren pH-Wert mittels HCl auf 6±0,5 eingestellt wird. Die Menge an freigesetzten Fluoridionen wird bei 300 l/h Messgasstrom und 30 ml/h Absorptionslösungsstrom gemessen.

Als Messgas dient ein Gasstrom aus Umgebungsluft, der variable Mengen an Hexafluorbenzol (0,5-5 ppm) enthält. Die gemessene Menge an freigesetzten Fluoridionen wird kontinuierlich potentiometrisch bestimmt.

## Patentansprüche

1. Verfahren zur kontinuierlichen, quantitativen Bestimmung von fluorhaltigen Verbindungen in Gasen, dadurch gekennzeichnet, dass man das Gas, das die fluorhaltige Verbindung enthält, mit einer wässrigen Lösung eines Amines behandelt und die Konzentration der abgespaltenen Fluoridionen mit einer direktpotentiometrischen Methode bestimmt.

2. Verfahren gemäss Anspruch 1, dadurch gekennzeichnet, dass die fluorhaltige Verbindung HF oder eine organische Fluorverbindung ist, bei der das Fluoratom direkt an einen aromatischen Ring gebunden ist.

3. Verfahren gemäss Anspruch 2, dadurch gekennzeichnet, dass die organische Fluorverbindung bei 20-35°C und unter Normaldruck flüssig oder gasförmig ist.

4. Verfahren gemäss Anspruch 3, dadurch gekennzeichnet, dass die organische Fluorverbindung 2,4,6-Trifluor-1,3,5-triazin ist.

5. Verfahren gemäss Anspruch 1, dadurch gekennzeichnet, dass das Verhältnis zwischen Messgas- und Absorptionslösungsstrom von 1000:1 bis 50000:1 beträgt.

6. Verfahren gemäss Anspruch 5, dadurch gekennzeichnet, dass das Verhältnis zwischen Messgas- und Absorptionslösungsstrom etwa 20000:1 beträgt.

7. Verfahren gemäss Anspruch 1, dadurch gekennzeichnet, dass das Amin in einer Menge von 0,01 bis 1 mol/l in der wässrigen Lösung enthalten ist.

8. Verfahren gemäss Anspruch 7, dadurch gekennzeichnet, dass das Amin in einer Menge von 0,05 bis 0,3 mol/l in der wässrigen Lösung enthalten ist.

9. Verfahren gemäss Anspruch 1, dadurch gekennzeichnet, dass man als Amin ein primäres oder sekundäres C₁-C₂₀-Amin verwendet.

10. Verfahren genäss Anspruch 9, dadurch gekennzeichnet, dass man als Amin Diethylentriamin, Prolin, Morpholin, Piperazin oder Phenylendiamin verwendet.

11. Verfahren gemäss Anspruch 10, dadurch gekennzeichnet, dass man als Amin Piperazin verwendet.

12. Verfahren gemäss Anspruch 1, dadurch gekennzeichnet, dass die wässrige Absorptionslösung zusätzlich noch einen Elektrolyten, ein Tensid, eine Mineralsäure oder eine Mischung der genannten Stoffe enthält.

13. Mittel zur Freisetzung von Fluoridionen aus einer fluorhaltigen Substanz enthaltend 0,01-1 mol/l eines Amins, eine geringe Menge einer fluorhaltigen Referenzverbindung und Wasser, wobei der pH-Wert der Lösung in einem Bereich von 6±0,5 liegt.

14. Mittel gemäss Anspruch 13 enthaltend neben Wasser 0,05 bis 0,3 mol/l Piperazin, etwa 10⁻⁴ mol/l Natriumfluorid, wobei der pH-Wert mit Salzsäure auf 5,8 eingestellt ist.

15. Verwendung einer Lösung enthaltend 0,01-1 mol/l eines Amins zur Freisetzung von Fluoridionen aus einer fluorhaltigen Substanz.

16. Verwendung einer Lösung gemäss Anspruch 15, dadurch gekennzeichnet, dass als Amin Diethylentriamin, Prolin, Morpholin, Piperazin oder Phenylendiamin verwendet wird.

17. Verwendung einer Lösung genäss Anspruch 15 enthaltend neben Wasser 0,05 bis 0,3 mol/l Piperazin, etwa 10⁻⁴ mol/l Natriumfluorid, wobei der pH-Wert mit Salzsäure auf 5,8 eingestellt ist.
